# EUROPEAN PATENT SPECIFICATION

(11) **EP 1 119 304 B1**
(45) Date of publication and mention of the grant of the patent: **07.12.2005**
(21) Application number: 99951705.5
(22) Date of filing: 04.10.1999
(51) Int. Cl.: A61B 17/70

(54) **DEVICE FOR SECURING SPINAL RODS**
VORRICHTUNG ZUR BEFESTIGUNG VON WIRBELSÄULENSTANGEN
DISPOSITIF DE FIXATION DE BROCHES SPINALES

(30) Priority: 06.10.1998 US 167439
(43) Date of publication of application: 01.08.2001
(73) Proprietor: Howmedica Osteonics Corp., Mahwah, NJ 07430 (US)
(72) Inventor: YUAN, Hansen, Fayetteville, NY 13066 (US)
(74) Representative: Liebetanz, Michael
(86) International application number: PCT/US1999/022860
(87) International publication number: WO 2000/019923

(56) References cited:
- EP-A- 0 535 623
- DE-U- 9 403 231
- US-A- 5 562 663

## Description

The subject disclosure relates to implantable spinal stabilization systems for surgical treatment of spinal disorders, and more particularly, to a device for connecting cylindrical spinal rods of a spinal stabilization system to the spine according to the preamble of claim 1.

### 1. Background of the Related Art

The spinal column is a complex system of bones and connective tissue which protects critical elements of the nervous system. Despite these complexities, the spine is a highly flexible structure, capable of a high degree of curvature and twist through a wide range of motion. Trauma or developmental irregularities can result in spinal pathologies which limit this range of motion.

For many years, orthopedic surgeons have attempted to correct spinal irregularities and restore stability to traumatized areas of the spine through immobilization. Over the past ten years, spinal implant systems have been developed to achieve immobilization. Examples of such systems are disclosed in U.S. Patent Nos. 5,102,412 and 5,181,917 to Rogozinski. Such systems often include spinal instrumentation having connective structures such as elongated rods which are placed on opposite sides of the portion of the spinal column intended to be immobilized. Screws and hooks are commonly utilized to facilitate segmental attachment of such connective structures to the posterior surfaces of the spinal laminae, through the pedicles, and into the vertebral bodies. These components provide the necessary stability both in tension and compression to achieve immobilization.

Various fastening mechanisms have been provided in the prior art to facilitate securement of screws and hooks to the connective structures of a spinal stabilization system. For example, U.S. Patent No. 5,257,993 to Asher discloses an apparatus for use in retaining a spinal hook on an elongated spinal rod. The apparatus includes a body extending upwardly from a hook portion and having an open ended recess for receiving a spinal rod and an end cap engageable with the body to close the recess. A set screw is disposed in the center of the end cap to clamp the rod in the recess of the body. The end cap and body are interconnectable by different types of connectors including a bayonet connector, a linear cam connector or a threaded connector. Other examples of fastening mechanism for facilitating attachment of screws and hooks to the connective structures of a spinal stabilization system are disclosed in U.S. Patent No. 5,437,669 to Yuan et al. and U.S. Patent No. 5,437,670 to Sherman et al. U.S. patent 5,562,663 discloses another device with a theaded fastener comprising the features according to the preamble of claim 1. German Utility model DE 94 03 231 U1 discloses an external sleeve cap with an internal threaded fastener.

In each of these prior art examples, threaded fasteners are used to facilitate securement of the connector to the spinal rod. Yet it is well known that threaded fasteners can become loosened under the influence of cyclically applied loads commonly encountered by the spinal column. Furthermore, during assembly, excessive torque applied to a threaded fastener can cause damage to the fastener as well as to the connective device with which it is associated.

It would be beneficial to provide a more reliable and effective mechanism for facilitating the attachment of screws, hooks and clamps to the connective structures of a spinal stabilization system.

### SUMMARY OF THE DISCLOSURE

The subject disclosure is directed to a device for securing a spinal rod to a fixation device such as a pedicle screw or a lamina hook. The device disclosed herein includes a head portion configured to receive a spinal rod, a locking cap configured to engage the head portion and the spinal rod upon rotation of the locking cap relative to the head portion to secure the position of the head portion relative to the spinal rod, and a fastener portion extending from the head portion and configured to engage the spine. The fastener portion of the device can be in the form of a screw, hook or clamp, or any other configuration known in the art.

The head portion of the device has a channel extending therethrough for receiving a spinal rod and the channel is preferably bounded by opposed side walls each having an arcuate engagement slot defined therein. The locking cap preferably has opposed arcuate engagement flanges configured for reception in the opposed arcuate engagement slots of the head portion upon rotation of the locking cap relative to the head portion. Preferably, the opposed engagement slots are each defined in part by inclined slot surfaces, with the angle of the inclined surface of one engagement slot being opposite that of the opposed engagement slot. Similarly, the opposed engagement flanges are preferably each defined in part by inclined flange surfaces, with the angle of the inclined surface of one engagement flange being opposite that of the opposed engagement flange. The head portion also preferably includes structure for interacting with the locking cap to prevent the opposed side walls of the head portion from expanding radially outwardly when the arcuate flanges are engaged in the arcuate slots.

Preferably, the locking cap of the device is configured for rotation between an initial position in which the arcuate engagement flanges are 90° out of phase with the arcuate engagement slots, an intermediate position in which the arcuate engagement flanges are 45° out of phase with the arcuate engagement slots, and a locked position in which the arcuate engagement flanges are in phase and intimately engaged with the arcuate engagement slots.

In this regard, the bottom surface of the locking cap preferably includes a first recess oriented to accommodate a spinal rod when the locking cap is in an initial unlocked position, a second recesses which intersects the first recess at a first angle to accommodate a spinal rod when the locking cap is in an intermediate position, and a third recess which intersects the elongate recess at a second angle to accommodate a spinal rod when the locking cap is in a final locked position. In accordance with a preferred embodiment of the subject disclosure, the first recess is an elongate recess, the second recess is a transverse recess which intersects the elongate recess at a 45° angle, and the third recess is an orthogonal recess which intersects the elongate recess at a 90° angle.

These and other unique features of the device disclosed herein and the method of installing the same will become more readily apparent from the following description of the drawings.

### BRIEF DESCRIPTION OF THE DRAWINGS

So that those having ordinary skill in the art to which the disclosed apparatus appertains will more readily understand how to construct and use the same, reference may be had to the drawings wherein:
Fig. 1 is a perspective view of an elongated spinal rod of a spinal stabilization system having attached thereto a bone screw and a bone hook constructed in accordance with a first embodiment of the subject disclosure;
Fig. 2 is a perspective view of a locking cap which forms part of the bone screw and bone hook illustrated in Fig. 1, oriented in an inverted position for ease of illustration;
Fig. 3 is a perspective view of the bone screw and locking cap of Fig. 1 separated from one another for ease of illustration;
Fig. 4 is a cross-sectional view of the bone screw of the subject disclosure taken along line 4-4 of Fig. 1;
Fig. 5 is a cross-sectional view of the locking cap taken along line 5-5 of Fig. 3;
Figs. 6A through 6D illustrate operative steps associated with attaching the bone fastener of the subject disclosure to a spinal rod, wherein:

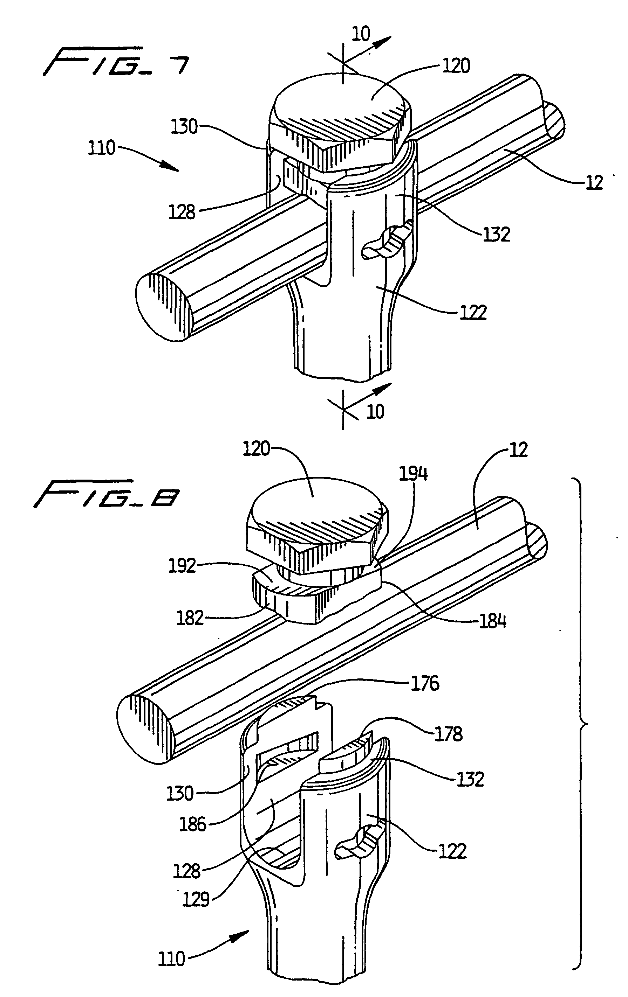

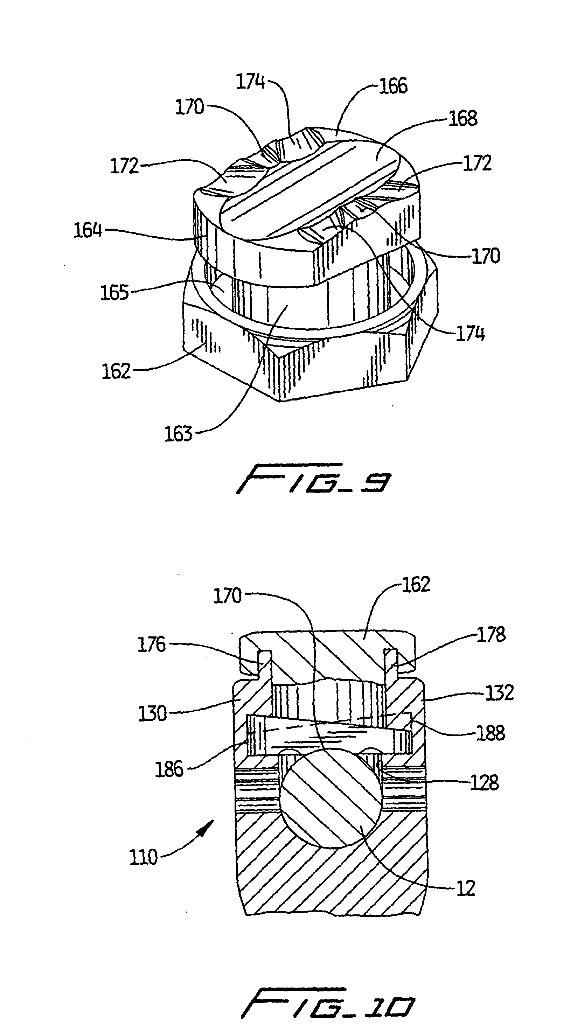

Fig. 6A illustrates the step of positioning the spinal rod and locking cap in the reception channel of the head portion of a fastening device of the subject disclosure;
Fig. 6B illustrates the initial orientation of the locking cap relative to the head portion of a fastening device of the subject disclosure wherein the locking cap is in an unlocked position;
Fig. 6C illustrates the rotation of the locking cap relative to the head portion of a fastening device of the subject disclosure to a partially locked position; and
Fig. 6D illustrates the rotation of the locking cap relative to the head portion of a fastening device of the subject disclosure to a locked position;
Fig. 7 is a perspective view of a fastening device constructed in accordance with a second embodiment of the subject disclosure;
Fig. 8 is a perspective view of the fastening device of Fig. 7 with the locking cap separated for ease of illustration;
Fig. 9 is a perspective view of the locking cap of the fastener device of Fig. 7, oriented in an inverted position for ease of illustration; and
Fig. 10 is a cross-sectional view of the fastening device of Fig. 7 taken along line 10-10 of Fig. 7.

These and other features of the apparatus disclosed herein will become more readily apparent to those having ordinary skill in the art from the following detailed description of the preferred embodiments taken in conjunction with the drawings.

### DETAILED DESCRIPTION OF THE PREFERRED EMBODIMENTS

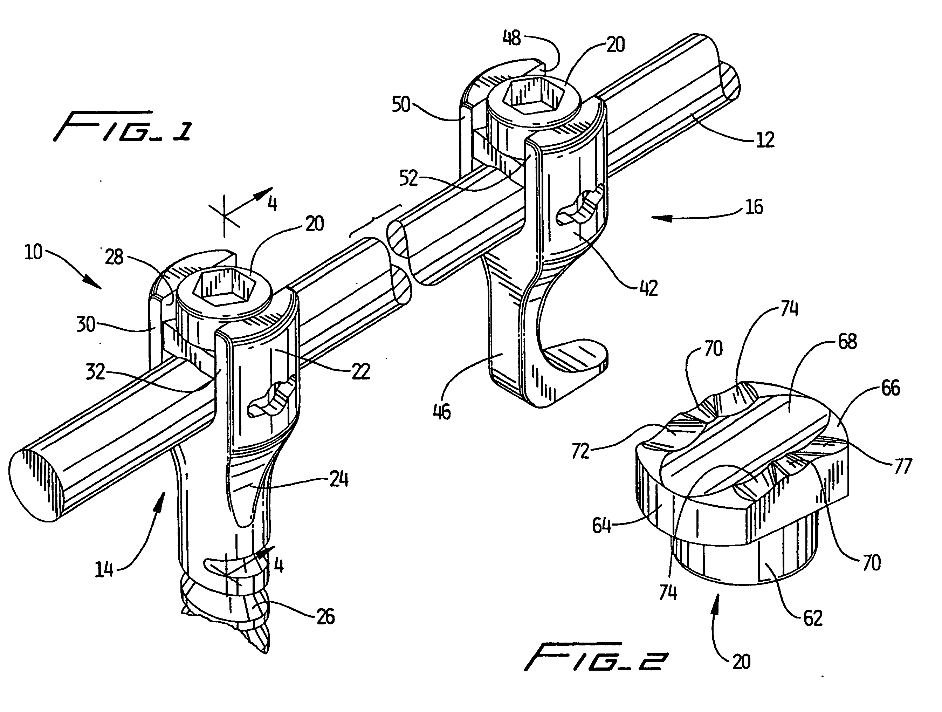

Referring now to the drawings wherein like reference numerals identify similar structural elements of the subject apparatus, there is illustrated in Fig. 1 a section of a spinal stabilization system constructed in accordance with a preferred embodiment of the subject disclosure and designated generally by reference numeral 10.

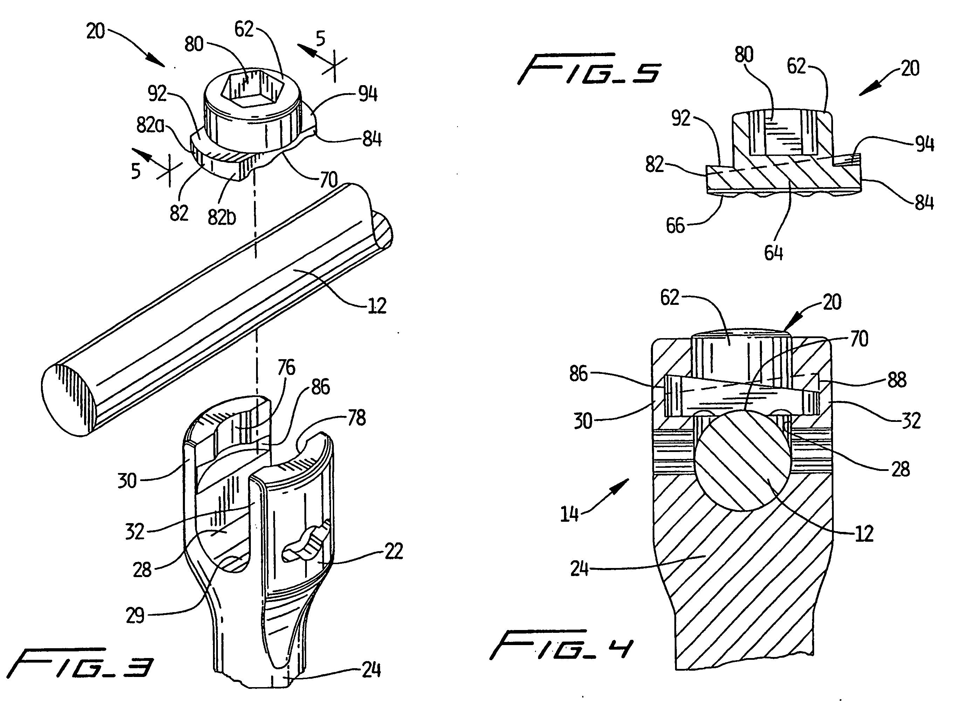

Referring to Fig. 1, spinal stabilization system 10 includes an elongated spinal rod 12 having a circular cross-section and a substantially smooth outer surface finish. As illustrated, fastening devices in the form of a bone screw 14 and right-angle hook 16 are provided for securing spinal rod 12 to the spine during a spinal stabilization procedure. Both fastening devices employ a novel top-loaded locking cap, designated generally by reference numeral 20, which will be described in greater detail hereinbelow with reference to Fig. 2. The novel locking cap achieves significant clinical advantages over the prior art through its reliability and the ease in which it is installed during a spinal stabilization procedure.

It should be recognized that the subject disclosure is not limited in any way to the illustrated bone screw and right-angle hook. Rather, these particular fasteners are merely examples of the type of devices that can employ the novel locking cap disclosed herein. Other fasteners commonly utilized in spinal stabilization systems, such as, for example, hooks having alternative angular geometries as well as clamps are also envisioned. Indeed, it is envisioned that any component designed for attachment to an elongated spinal rod or transverse coupling rod, may incorporate the novel locking cap of the subject disclosure. Also, any number of fastening devices can be applied along the length of the spinal rod.

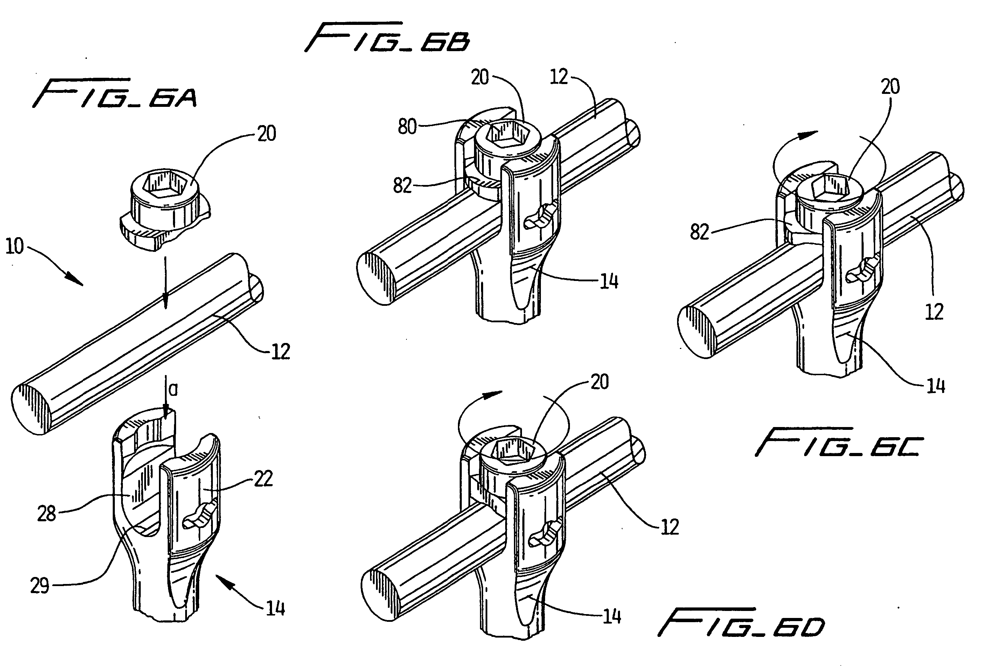

With continuing reference to Fig. 1, bone screw 14 includes a head portion 22 defining a horizontal axis and a vertical axis. A shank portion 24 depends from the head portion and a threaded portion 26 having a helical thread extending about the outer periphery depends from the shank portion. The helical thread is particularly adapted to securely engage the vertebral bodies of the spine. A channel 28 extends through the head portion 22 along the horizontal axis thereof for receiving elongated spinal rod 12. As best seen in Fig. 3, channel 28 is defined by the interior surfaces of side walls 30 and 32 and the curved lower surface 29 which extends therebetween. Locking cap 20 is dimensioned and configured for reception and engagement in locking channel 28 to secure the position of bone screw 14 with respect to spinal rod 12 during a spinal stabilization procedure.

Referring again to Fig. 1, right-angle hook 16 includes a head portion 42 defining a horizontal axis and a vertical axis. A hook portion 46 depends from the head portion 42 for securement to a vertebral body of the spine. A channel 48 extends through the head portion 22 along the horizontal axis thereof for receiving elongated spinal rod 12. Channel 48 is defined by the interior surfaces of opposed side walls 50 and 52 and a curved lower surface extending therebetween. Locking cap 20 is dimensioned and configured for reception and engagement in channel 48 to secure the position of hook 16 with respect to spinal rod 12 during a spinal stabilization procedure.

Referring now to Fig. 2, there is illustrated locking cap 20 in an inverted position to best illustrate structural aspects thereof. Locking cap 20 includes a cylindrical head 62 and a flanged portion 64. The bottom surface 66 of flanged portion 64 includes an elongate recess 68 having a curvature complementary to spinal rod 12 for accommodating the spinal rod when locking cap 20 is in an unlocked position, shown for example in Fig. 6B. In such a position, the fastening device may be moved freely along or rotated about the longitudinal axis of the spinal rod. Bottom surface 66 also includes a bifurcated orthogonal recess 70 which intersects the elongate recess at a 90° angle and has a curvature complementary to spinal rod 12 to accommodate the spinal rod when locking cap 20 is in a locked position, shown for example in Fig. 6D and Fig. 4. In addition, bottom surface 66 includes bifurcated first and second transverse recesses 72 and 74 which intersect the elongate recess 68 at opposite angles of intersection and have curvatures which are complementary to spinal rod 12 to accommodate the spinal rod when the locking cap 20 is in either of two intermediate positions, one of which is shown for example in Fig. 6C. In such a position, the fastening device retains the spinal rod but is not fully secured, and if desired by the surgeon, locking cap 20 can be rotated from the intermediate position and the fastener moved to an alternative location on the spinal rod. Preferably, the transverse recesses intersect the elongate recess at opposed 45° angles. However, those skilled in the art will readily appreciate that the transverse recess can be oriented at alternative intersecting angles. It is also contemplated that the bottom surface can be flat without any recesses.

Referring to Figs. 3 and 5, the cylindrical head 62 of locking cap 20 includes a hexagonal axial bore 80 extending partially therethrough for receiving a working implement such as a wrench to facilitate rotation of the locking cap 20 relative to the head portion 22 of the fastening device about the vertical axis defined thereby. It envisioned that alternative tooling configurations known in the art can also be utilized to facilitate axial rotation of locking cap 20 during a surgical procedure. Curved notches 76 and 78 are formed in the inner surfaces of opposed walls 30 and 32 for accommodating the cylindrical head 62 of locking cap 20 when the locking cap is received and rotated within channel 28.

The flanged portion 64 of locking cap 20 is defined in part by two diametrically opposed arcuate engagement flanges 82 and 84 which are dimensioned and configured for operative engagement with two complementary diametrically opposed arcuate engagement slots 86 and 88 defined in the interior surfaces of the opposed side walls 30 and 32 of head portion 22. (See Fig. 4).

With continuing reference to Figs. 3 through 5, engagement flanges 82 and 84 define ramped camming surfaces 92 and 94, respectively. Camming surfaces 92 and 94 are of opposite angular inclination with respect to one another. More particularly, each engagement flange has a low side (e.g., 82a of flange 82) and a high side (e.g., 82b of flange 82), whereby the low sides of the two flanges are diametrically opposed from one another as are the high sides. Actually, the camming surfaces of the flanges are mirror images of one another. Thus, the locking cap can be initially oriented with either flange aligned to engage either slot. This versatility adds to the ease in which the locking cap is installed during a surgical procedure.

As best seen in Fig. 4, the arcuate engagement slots 86 and 88 in head portion 22 of fastener 14 have inclined surfaces which mate with the ramped camming surfaces 92 and 94 of flanges 82 and 84. As best seen in Fig. 5, the ramped camming surfaces 92 and 94 are tapered radially inwardly to enhance the interlock with the mating surfaces of arcuate engagement slots 86 and 88, which are also tapered to complement the radially inward taper of camming surfaces 92 and 94. This interlocking relationship serves to prevent the opposed side walls 30 and 32 of head portion 22 from spreading radially outward as the arcuate flanges are engaged with the arcuate slots when the locking cap 20 is rotated to a locked position.

Figs 6A through 6D illustrate the steps in securing the fastening device to the spinal rod during a surgical procedure. Although attachment of a bone screw 14 is shown, it should be understood, as noted above, that other fastening devices, e.g., bone hooks, can be secured to the spinal rod 12 using the locking cap and head portion structure of the present disclosure. Initially, as illustrated in Fig. 6A, spinal rod 12 is moved into approximation with the horizontal channel 28 of head portion 22 such that the periphery of the spinal rod 12 is in registration with the curved surface 29 of the channel 28. Locking cap 20 is then top loaded into the channel along the vertical axis of the fastener in the direction of arrow a. At such a time, spinal rod 12 is accommodated within the elongate recess 68 defined in the bottom surface 66 of locking cap 20 and the bone screw 14 may be moved freely relative to the spinal rod. The opposed flanged sections 82 and 84 of locking cap 20 are 90° out of phase from the opposed arcuate engagement slots 86 and 88 defined in head portion 22, as shown for example in Fig. 6B.

Thereafter, as shown in Fig. 6C, locking cap 20 is rotated 45° relative to head portion 22 about the vertical axis thereof. At such a time, spinal rod 12 is accommodated within one of the two transverse recesses 72 or 74, depending upon the initial orientation of the locking cap 20 with respect to the head portion. Thereupon, the opposed arcuate engagement flanges 82 and 84 of locking cap 20 are only partially engaged with the opposed arcuate engagement slots 86 and 88 defined in head portion 22, as they are 45° out of phase with the slots. Consequently, the locking cap holds the fastener 22 and spinal rod 12 together, but does not lock the fastener. In this position, the locking cap 20 can be readily rotated in the opposite direction to disengage from the spinal rod 12 to adjust the position of the bone screw 14 with respect to the spinal rod 12.

Once the desired position and orientation of the bone screw 14 has been attained, locking cap 20 is rotated another 45° to the locked position illustrated in Fig. 6D. At such a time, spinal rod 12 is accommodated within the orthogonal recess 70 defined in the bottom surface of locking cap 20. Thereupon, the opposed engagement flanges 82 and 84 of flanged portion 64 are fully engaged with the opposed engagement slots 86 and 88 of head portion 22, and the longitudinal and angular orientations of the bone screw 14 are fixed with respect to spinal rod 12, as illustrated in Fig. 4. It should be readily apparent that the manner and method by which bone screw 14 hook is attached to spinal rod 12 is identical to the manner and method by which hook 16 or other fasteners are attached to spinal rod 12.

Since the rotational range of locking cap 20 is limited, i.e., the locking cap can only be rotated 90°, it will be readily appreciated that the cap cannot be over-torqued. Thus, the damage often caused by over-tightening a conventional threaded locking mechanism, such as a set screw, is avoided. Furthermore, since the locking cap of the subject disclosure has a predetermined locked position, it is unlikely that it will be under-torqued or left in a loose condition after installation as is common with threaded set screws found in the prior art. That is, by having a predetermined locked position, uniform locking forces are provided for all of the fastening devices used to secure the spinal rod 12 along its length and cross threading is reduced.

Referring now to Figs. 7 and 8, there is illustrated another fastening device constructed in accordance with a preferred embodiment of the subject disclosure and designated generally by reference numeral 110. Fastening device 110 is similar to fastening devices 12 and 14 in that it is particularly designed to facilitate securement of a spinal rod to the spine in a convenient manner. Fastening device 110 includes a head portion 122 having opposed side walls 130 and 132 which define a horizontal channel 128 in conjunction with the curved lower surface 129 extending therebetween. Arcuate tabs 176 and 178 project upwardly from side walls 130 and 132, respectively, for interacting with locking cap 120.

Referring to Fig. 9, locking cap 120, which is shown in an inverted position for ease of illustration, includes a hexagonal head 162, a cylindrical body 163 and a flanged portion 164. The hexagonal head 162 is adapted and configured for interaction with a wrench or similar work implement. An annular channel 165 extends into the bottom surface of hexagonal head 162 for receiving arcuate tabs 176 and 168. This positive interaction serves to prevent the opposed side walls 130 and 132 of head portion 122 from spreading radially outwardly when arcuate flanges 182 and 184 of locking cap 120 are engaged in arcuate slots 186 and 188 of head portion 122 upon rotation of locking cap 20 into a locked position. Thus, in this embodiment, the ramped camming surfaces 192 and 194 of the arcuate engagement flanges 182 and 184 need not be provided with radially inwardly directed tapers as provided on flanges 82 and 84 of the locking cap 20 of the embodiment of Figs. 1-6.

With continuing reference to Fig. 9, the bottom surface 166 of the flanged portion 164 of locking cap 120 is configured in substantially the same manner as the bottom surface 66 of locking cap 20 in that it is provided with an elongate recess 168 for accommodating a spinal rod when the locking cap 120 is in an unlocked position, first and second bifurcated transverse recesses 172 and 174 which intersect the elongate recess 168 at opposite 45° angles to accommodate the spinal rod when the locking cap 120 is in either of two intermediate positions, and a bifurcated orthogonal recess 170 which intersects the elongate recess at a 90° angle to accommodate the spinal rod when the locking cap 120 is in a final locked position, as shown in Fig. 10. It will be readily appreciated that locking cap 120 is engaged with fastening device 110 in a manner that is substantially similar to the manner in which locking cap 20 is engaged with bone fastener 14 and hook 16, and that the configuration of the bottom surface of flanged portion 164 provides the same benefits afforded by the flanged portion 64 of locking cap 20.

Although the apparatus disclosed herein has been described with respect to preferred embodiments, it is apparent that modifications and changes can be made thereto without departing from the scope of the invention as defined by the claims.

## Claims

1. A device for securing a spinal rod (12) to the spine comprising:
- a head portion (22; 42) having a channel (28; 48) therethrough for receiving the spinal rod (12),
- a locking cap (20) to engage the head portion (22; 42) and the spinal rod (12), and
- a fastener portion (14, 16) extending from the head portion (22; 42) and to engage the spine,
wherein the channel (28; 48) is bounded by opposite side walls (30, 32; 50, 52) and each of the opposed side walls (30, 32; 50, 52) has an arcuate engagement slot (86, 88) defined therein and wherein the locking cap (20) has opposed arcuate engagement flanges (82, 84) configured for reception in the opposed arcuate engagement slots (86, 88) of the head portion (22; 42) upon rotation of the locking cap (20) relative to the head portion (22; 42),
**characterized in that** said arcuate engagement slot (86, 88) is curved about an axis being perpendicular to said channel (28, 48) and lying in a plane that is oriented between the opposite side walls (30, 32; 50, 52) to secure the position of the head portion (22; 42) relative to the spinal rod (12) upon rotation of the locking cap (20) relative to the head portion (22; 42) around said axis.

2. The device according to claim 1, wherein the locking cap (20) is configured for rotation between an initial position in which the arcuate engagement flanges (82, 84) are 90° out of phase with the arcuate engagement slots (86, 88), an intermediate position in which the arcuate engagement flanges (82, 84) are 45° out of phase with the arcuate engagement slots (86, 88) and a locked position in which the arcuate engagement flanges (82, 84) are in phase and intimately engaged with the arcuate engagement slots (86, 88).

3. The device according to claim 1 or 2, wherein the bottom surface (66) of the locking cap (20) includes an elongate recess (68) oriented to accommodate the spinal rod (12) when the locking cap (20) is in an initial position.

4. The device according to claim 3, wherein the bottom- surface (66) of the locking cap (20) includes an orthogonal recess (70) which intersects the elongate recess (68) at a 90° angle to accommodate the spinal rod (12) when the locking cap (20) is in a locked position.

5. The device according to claim 3 or 4, wherein the bottom surface (66) of the locking cap (20) includes at least one traverse recess (72, 74) which intersects the elongate recess (68) at a 45° angle to accommodate the spinal rod (12) when the locking cap (20) is in a intermediate position.

6. The device according to one of the preceding claims, wherein the locking cap (20) has a cylindrical head (62) which includes a hexagonal bore (80) for receiving a work implement.

7. The device according to one of the preceding claims 1 to 5, wherein the locking cap (20) has a hexagonal head (120) configured for reception by a work implement.

8. The device according to one of the preceding claims 1 to 7, wherein the engagement flanges (82, 84) define ramped camming surfaces (92, 94) of opposite angular inclination with respect to one another and wherein the arcuate engagement slots (86, 88) have inclined surfaces which mate with the ramped camming surfaces (92, 94) of flanges (82, 84).

9. The device according to claim 8, wherein the ramped camming surfaces (92, 94) are tapered radially inwardly and wherein the arcuate engagement slots (86, 88) are tapered to complement the radially inward taper of camming surfaces (92, 94) to enhance the interlock of the mating surfaces.

## Patentansprüche

1. Vorrichtung zur Befestigung von einer Wirbelsäulenstange (12) an der Wirbelsäule:
- mit einem Kopfabschnitt (22; 42) mit einem durch diesen hindurchgehenden Kanal (28; 48) zur Aufnahme der Wirbelsäulenstange (12),
- mit einer Verschlusskappe (20), um in den Kopfabschnitt (22; 42) und die Wirbelsäulenstange (12) einzugreifen, und
- mit einem Befestigungsabschnitt (14, 16), der sich von dem Kopfabschnitt (22; 42) wegerstreckt, um in die Wirbelsäule einzugreifen,
wobei der Kanal (28; 48) von gegenüberliegenden Seitenwänden (30, 32; 50, 52) begrenzt ist und jede der gegenüberliegenden Seitenwände (30, 32; 50, 52) einen gebogenen Eingriffsschlitz (86, 88) aufweist, der darin definiert ist, und wobei die Verschlusskappe (20) gegenüberliegende gebogene Eingriffsflansche (82, 84) aufweist, die so ausgestaltet sind, um in den gegenüberliegenden gebogenen Eingriffsschlitzen (86, 88) des Kopfabschnitts (22; 42) bei Drehung der Verschlusskappe (20) relativ zum Kopfabschnitt (22; 42) aufgenommen zu werden,
**dadurch gekennzeichnet, dass** der besagte gebogene Eingriffsschlitz (86, 88) um eine Achse gebogen ist, die senkrecht zu dem besagten Kanal (28, 48) ist und in einer Ebene liegt, die zwischen den gegenüberliegenden Seitenwänden (30, 32; 50, 52) orientiert ist, um die Position des Kopfabschnittes (22; 42) relativ zur Wirbelsäulenstange (12) bei Drehung der Verschlusskappe (20) relativ zum Kopfabschnitt (22; 42) um die besagte Achse sicherzustellen.

2. Vorrichtung nach Anspruch 1, bei der die Verschlusskappe (20) für eine Rotation ausgestaltet ist zwischen einer anfänglichen Position, in der die gebogenen Eingriffsflansche (82, 84) um 90° aus der Phase mit den gebogenen Eingriffsschlitzen (86, 88) sind, einer Zwischenposition, bei der die gebogenen Eingriffsflansche (82, 84) um 45° aus der Phase mit den gebogenen Eingriffsschlitzen (86, 88) sind, und einer Verschlussposition, bei der die gebogenen Eingriffsflansche (82, 84) in Phase und direkt im Eingriff mit den gebogenen Eingriffsschlitzen (86, 88) stehen.

3. Vorrichtung nach Anspruch 1 oder 2, bei der die Bodenoberfläche (66) der Verschlusskappe (20) eine längliche Ausnehmung (68) umfasst, die so orientiert ist, um die Wirbelsäulenstange (12) aufzunehmen, wenn die Verschlusskappe (20) in einer anfänglichen Position ist.

4. Vorrichtung nach Anspruch 3, bei der die Bodenoberfläche (66) der Verschlusskappe (20) eine orthogonale Ausnehmung (70) umfasst, die die längliche Ausnehmung (68) in einen 90° Winkel schneidet, um die Wirbelsäulenstange (12) aufzunehmen, wenn die Verschlusskappe (20) in einer Verschlussposition ist.

5. Vorrichtung nach Anspruch 3 oder 4, bei der die Bodenoberfläche (66) der Verschlusskappe (20) mindestens eine querverlaufende Ausnehmung (72, 74) umfasst, die die längliche Ausnehmung (68) in einen 45° Winkel schneidet, um die Wirbelsäulenstange (12) aufzunehmen, wenn die Verschlusskappe (20) in einer Zwischenposition ist.

6. Vorrichtung nach einem der vorstehenden Ansprüche, bei der die Verschlusskappe (20) einen zylindrischen Kopf (62) aufweist, der eine hexagonale Bohrung (80) umfasst, um ein Werkzeughilfsmittel aufzunehmen.

7. Vorrichtung nach einem der vorstehenden Ansprüche 1 bis 5, bei der die Verschlusskappe (20) einen hexagonalen Kopf (120) aufweist, der für die Aufnahme eines Werkzeughilfsmittels ausgestaltet ist.

8. Vorrichtung nach einem der vorstehenden Ansprüche 1 bis 7, bei der die Eingriffsflansche (82, 84) rampenförmige Nockenoberflächen (92, 94) von zueinander entgegengesetzt ausgerichteten Winkelneigungen definieren, und bei der die gebogenen Eingriffsschlitze (86, 88) geneigte Oberflächen aufweisen, die mit den rampenförmigen Nockenoberflächen (92, 94) der Flansche (82, 84) übereinstimmen.

9. Vorrichtung nach Anspruch 8, bei der die rampenförmigen Nockenoberflächen (92, 94) radial nach innen verjüngt ausgestaltet sind und bei der die gebogenen Eingriffsschlitze (86, 88) verjüngt ausgestaltet sind, um zu den radial nach innen verjüngt ausgeführten Nockenoberflächen (92, 94) komplementär ausgestaltet zu sein, um den Eingriff der übereinstimmenden Oberflächen zu verbessern.

## Revendications

1. Dispositif de fixation d'une broche spinale (12) sur la colonne vertébrale comprenant :
- une portion de tête (22 ; 42) présentant un canal (28 ; 48) au travers de celle-ci afin de recevoir la broche spinale (12),
- un capuchon de blocage (20) destiné à venir en prise avec la portion de tête (22 ; 42) et la broche spinale (12), et
- une portion de fixation (14, 16) s'étendant à partir de la portion de tête (22 ; 42) et destinée à venir en prise avec la colonne vertébrale,
dans lequel le canal (28 ; 48) est limité par des parois latérales opposées (30, 32 ; 50, 52) et chacune des parois latérales opposées (30, 32 ; 50, 52) possède une fente de mise en prise arquée (86, 88) définie à l'intérieur de celle-ci et dans laquelle le capuchon de blocage (20) a des brides de mise en prise arquées opposées (82, 84) configurées pour être reçues dans les fentes de mise en prise arquées opposées (86, 88) de la portion de tête (22 ; 42) lors de la rotation du capuchon de blocage (20) par rapport à la portion de tête (22 ; 42),
**caractérisé en ce que** ladite fente de mise en prise arquée (86, 88) est incurvée autour d'un axe qui est perpendiculaire audit canal (28, 48) et se situe dans un plan qui est orienté entre les parois latérales opposées (30, 32 ; 50, 52) afin de fixer la position de la portion de tête (22 ; 42) par rapport à la broche spinale (12) lors de la rotation du capuchon de blocage (20) par rapport à la portion de tête (22 ; 42) autour dudit axe.

2. Dispositif selon la revendication 1, dans lequel le capuchon de blocage (20) est configuré pour une rotation entre une position initiale dans laquelle les brides de mise en prise arquées (82, 84) sont décalées de 90° par rapport aux fentes de mise en prise arquées (86, 88), une position intermédiaire dans laquelle les brides de mise en prise arquées (82, 84) sont décalées de 45° par rapport aux fentes de mise en prise arquées (86, 88) et une position de blocage dans laquelle les brides de mise en prise arquées (82, 84) sont alignées et en prise étroite avec les fentes de mise en prise arquées (86, 88).

3. Dispositif selon la revendication 1 ou 2, dans lequel la surface inférieure (66) du capuchon de blocage (20) inclut un évidement allongé (68) orienté de façon à recevoir la broche spinale (12) lorsque le capuchon de blocage (20) est dans une position initiale.

4. Dispositif selon la revendication 3, dans lequel la surface inférieure (66) du capuchon de blocage (20) inclut un évidement orthogonal (70) qui coupe l'évidement allongé (68) sous un angle de 90° afin de recevoir la broche spinale (12) lorsque le capuchon de blocage (20) est dans une position de blocage.

5. Dispositif selon la revendication 3 ou 4, dans lequel la surface inférieure (66) du capuchon de blocage (20) inclut au moins un évidement transversal (72, 74) qui coupe l'évidement allongé (68) sous un angle de 45° afin de recevoir la broche spinale (12) lorsque le capuchon de blocage (20) est dans une position intermédiaire.

6. Dispositif selon l'une des revendications précédentes, dans lequel le capuchon de blocage (20) possède une tête cylindrique (62) qui inclut un orifice hexagonal (80) destiné à recevoir un outil de travail.

7. Dispositif selon l'une des revendications précédentes 1 à 5, dans lequel le capuchon de blocage (20) possède une tête hexagonale (120) configurée pour être reçue par un outil de travail.

8. Dispositif selon l'une des revendications précédentes 1 à 7, dans lequel les brides de mise en prise (82, 84) définissent des surfaces de taquets inclinées (92, 94) d'inclinaison angulaire opposée l'une par rapport à l'autre et dans lequel les fentes de mise en prise arquées (86, 88) ont des surfaces inclinées qui s'accouplent avec les surfaces de taquets inclinées (92, 94) des brides (82, 84).

9. Dispositif selon la revendication 8, dans lequel les surfaces de taquets inclinées (92, 94) sont biseautées radialement vers l'intérieur et dans lequel les fentes de mise en prise arquées (86, 88) sont biseautées afin de compléter le biseau radial vers l'intérieur des surfaces de taquets (92, 94) pour favoriser le blocage mutuel des surfaces d'accouplement.
